# EUROPEAN PATENT APPLICATION

(11) **EP 0 971 037 A2**
(43) Date of publication of application: **12.01.2000**
(21) Application number: 99305274.5
(22) Date of filing: 02.07.1999
(51) Int. Cl.: C12Q 1/32, C12Q 1/42

(54) **Stable reagent for formazan-based assay**

(30) Priority: 06.07.1998 US 110273
(71) Applicant: Kirkegaard & Perry Laboratories, Inc., Gaithersburg, MD 20879-4174 (US)
(72) Inventor: Hearl, William, Damascus, Maryland 20872 (US); Wright, Dennis, Tallahasse, Florida 32301 (US)
(74) Representative: Dean, John Paul

(57) **Abstract**

This invention relates to a stable, all-in-one aqueous reagent comprising 5-methyl-phenazinium methyl sulfate (phenazine methosulfate (PMS)), nitric acid and a metal salt wherein the metal is selected from the group consisting of vanadium, manganese, cobalt, and mixtures thereof, useful in assays which use alkaline phosphatase as a reporter enzyme and a reduction of a tetrazolium salt to a formazan as a detection/signaling system for detecting or quantifying a target substance in a sample.

## Description

### BACKGROUND OF THE INVENTION

### 1. Field of the Invention

This invention broadly relates to a stable aqueous reagent comprising an aqueous solution of 5-methyl-phenazinium methyl sulfate (phenazine methosulfate (PMS)), which is stable against discoloration. This reagent is useful for conducting both qualitative and quantitative assays for the presence of a target substance using a tetrazolium indicator. More particularly, the invention relates to a stable, all-in-one aqueous reagent useful, for example, in assays which use alkaline phosphatase as a reporter enzyme and a reduction of a tetrazolium salt to a formazan as a detection/signaling system for detecting or quantifying the presence of a target substance in a sample.

### 2. Description of Related Art

The detection or analysis of minute quantities of substances in biological and non-biological samples, both qualitative and quantitative, has become a routine practice in clinical, diagnostic and analytical laboratories. Such detection systems may be based on ligand-receptor interactions (e.g., immunoassay-based techniques), polynucleotide hybridizations and other chemical interactions.

These detection systems generally share one common feature, *i.e*., the use of an indicator material which signifies the presence of the target material in a sample through the use of colormetric, fluorometric, radiometric or other indicator techniques. One common colormetric indicator system relies upon the conversion (reduction) of a tetrazolium salt to a formazan, referred to hereinafter as a formazan-based assay. In these formazan-based assay systems, the presence of, or the formation of, a reducing agent (such as NADH) during the assay causes a reduction of a tetrazolium salt to a highly colored (and often insoluble) formazan species.

One of the recognized drawbacks of the formazan-based assay is that the reducing agent may not be strong enough to cause a rapid reduction of the tetrazolium salt to the formazan. As a result, the reduction of the reducible tetrazolium species can be undesirably slow. In such cases, the art has suggested using an electron transfer agent to facilitate the reduction of the tetrazolium salt to the formazan. Such electron transfer agents have been widely used and include the phenazines, such as phenazine methosulfate (PMS) and phenazine ethosulfate, the naphthazines, such as Roseinduline 2G, the phenothiazines, such as thionine, and the phenazonium compounds, such as Cresyl blue.

Formazan-based assays have been widely used for the histochemical detection of oxidative enzymes (where the tetrazolium salt is reduced by dehydrogenase enzymes to form the colored formazan); for the determination of creatine phosphokinase in body fluids (see U.S. Patent 4,012,286); for the determination of glutamate and glutamic transaminases (see U.S. Patent 4,024,021); for the determination of glycerol and triglycerides (see U.S. Patent 4,309,502); for the determination of cholesterol (see U.S. Patent 4,892,817); for the determination of serum fructosamine (see U.S. Patent 5,156,947); for the determination of ethanol (see Can. Patent 1,351,547); for the determination of creatinine (see U.S. Patent 4,215,197); and for the determination of lactic acid (see U.S. Patent 4,849,347).

More recently, Wright, in U.S. Patent 5,354,658, described a formazan-based immunoassay or nucleic acid hybridization assay which uses a solution of bromo-chloro-indolyl phosphate (BCIP), phenazine methosulfate (PMS) and dimethylthiazol diphenyl tetrazolium (MTT) as the color indicator system. The assay cascade is activated by the presence of alkaline phosphatase (AP) as a reporter enzyme.

One problem common to the acceptance and use of any of these systems, which rely on PMS as an electron transfer agent in the assay, is the inherent instability of PMS in an aqueous solution. It has long been recognized that in an aqueous solution, PMS undergoes a gradual decomposition to form a colored species. Indeed, it has been observed that long term storage of an aqueous solution of PMS causes a gradual and irreversible formation of a dark color. To avoid this storage problem, the operative reagents in some of the aforementioned assays are formulated as dry products in the form of tablets, dip-sticks and the like. The PMS solution is then freshly prepared before conducting the assay either using such tablets or using powdered PMS. The need to mix up fresh reagent each time an assay is conducted, however, introduces complexity into the assay and is considered a significant disadvantage to the widespread commercialization of this indicator system. The PMS instability often is so severe that its presence in the reagent limits incubation times, otherwise the color generated by PMS decomposition complicates any determination using the assay. It would be highly advantageous if PMS could be formulated as a color stable aqueous solution, particularly in combination with other components needed to carry out any particular assay.

Geisler et al., U.S. Patent 4,847,196 and U.S. Patent 4,613,569 indicate that solutions of tetrazolium salts can be stabilized against color-producing decomposition reactions by including one to ten moles of a complex forming acid such as boric acid or an organic hydroxypolycarboxylic acid, such as citric acid or malic acid, per mole of tetrazolium salt. These patents do not describe using PMS in the assay and the problem caused by the instability of PMS in aqueous solution is not addressed.

It is an object of this invention, therefore, to provide a novel reagent composition for use in a formazan-based assay.

It is another object of the invention to provide a composition in which an aqueous solution of PMS is stabilized against the formation of highly colored species.

It is yet another object of the invention to provide a stable, all-in-one aqueous reagent useful, for example, in assays which use alkaline phosphatase as a reporter enzyme and a reduction of a tetrazolium salt to a formazan as a detection/signaling system for detecting or quantifying a target substance in a sample.

It is still another object of the present invention to provide a novel reagent for use in a formazan-based assay to overcome one or more of the aforementioned disadvantages associated with the decomposition of PMS.

### DETAILED DESCRIPTION OF THE INVENTION

The present invention is directed to an aqueous reagent composition useful in conducting a formazan-based assay, wherein the reagent comprises an aqueous solution comprising PMS, nitric acid and a water soluble metal salt wherein the metal is selected from the group consisting of vanadium, manganese, cobalt and mixtures thereof.

More particularly, the invention relates to a stable, all-in-one aqueous reagent useful, for example, in assays which use alkaline phosphatase as a reporter enzyme and a reduction of a tetrazolium salt to a formazan as a detection/signaling system for detecting or quantifying a target substance in a sample, which reagent comprises an aqueous solution of PMS, nitric acid and a water soluble metal salt wherein the metal is selected from the group consisting of vanadium, manganese, cobalt, and mixtures thereof, in admixture with a dissolved tetrazolium salt. In preferred practice, the reagent further includes 5-bromo-4-chloroindoxyl phosphate (BCIP).

The aqueous reagent of the present invention is broadly useful in all formazan based-assays, including histological assays and assays based on ligand-receptor interactions, including antigen-antibody interactions and polynucleotide hybridizations. It has been observed that all-in-one reagents of the present invention retain a pale yellow color for extended periods of storage even at room temperature. In the absence of the nitric acid and specific water soluble metal salt, such reagents would become darkly colored very quickly.

The preferred all-in-one aqueous reagent of the present invention is particularly useful in an assay using an alkaline phosphatase (AP) conjugate as a reporter molecule or enzyme-label. In one embodiment, the AP enzyme is attached directly to a detector molecule or probe. The detector molecule or probe is the complementary immunological partner (antigen or antibody) of a particular target molecule (or fraction thereof) or the detector molecule or probe is a polynucleotide hybridization partner of a particular target molecule (or fraction thereof).

The present invention is based on the discovery that the presence, in an aqueous solution of PMS, of nitric acid and a dissolved salt of a metal selected from the group consisting of vanadium, manganese, cobalt and mixtures thereof stabilizes the PMS against the formation of a colored decomposition product.

The aqueous reagent of the present invention can be prepared by mixing together PMS, nitric acid and a water soluble metal salt, wherein the metal is selected from the group consisting of vanadium, manganese, cobalt, and mixtures thereof. The order of addition is not critical.

In addition to PMS, one essential component of the aqueous reagent composition of the present invention is nitric acid. The nitric acid can be supplied to the composition conveniently as a dilute aqueous solution containing from about 0.5 wt. % to 5 wt. % nitric acid based on the total weight of the diluted aqueous solution. Preferably, sufficient nitric acid is present in the aqueous reagent composition to provide from about 10 to 50 mols of nitric acid per mol of PMS.

The other essential component of the reagent composition is the dissolved metal salt. While the cationic metal species can be supplied as any water soluble metal salt, such as a metal chloride, metal sulfate, or metal phosphate, it is preferred to supply the metal as a water soluble nitrate salt. Preferably, sufficient water soluble metal salt is provided to yield from about 0.5 to about 2.0 mol of metal per mol of PMS in the reagent composition. Usually, the reagent solution will contain from about 2 to about 10 ppm of metal. One convenient way to supply both the nitric acid and the metal nitrate salt is to dissolve the elemental metal selected from vanadium; manganese, cobalt, and their mixtures in a dilute nitric acid solution. Adding the elemental metal to the dilute nitric acid solution up to a concentration of from about 500 to about 2000 ppm of the metal should be suitable in most instances. Because vanadium acts as a competitive inhibitor of alkaline phosphatase, cobalt and manganese generally are the metals of choice, used individually or in combination in the preferred formazan-based assay. Since the presence of a small amount of manganese helps to reduce the tendency of cobalt to precipitate, they preferably are used in combination. For example, 6ppm Mn may be used in combination with 2ppm Co. The manganese also is exchangeable with magnesium as a cofactor for alkaline phosphatase in the preferred assay system.

Exemplary aqueous solutions of cobalt, manganese, and/or vanadium include but are not limited to: (i) cobalt (2 ug/ml->200 ug/ml), (ii) manganese (2 ug/ml->200 ug/ml), (iii) vanadium (10 ug/ml->200 ug/ml), (iv) cobalt and manganese (2 ug/ml Co and 4ug/ml Mn). The exemplary reagent formulations using the aforementioned exemplary aqueous solutions of cobalt, manganese and/or vanadium have a pH range of 5.5 - 11.0. Outside this pH range, formazan may participate. Further, cobalt chloride is not preferred because CoCl₂ may not stabilize PMS. Accordingly, salts of cobalt other than CoCl₂ are preferred. Vanadium may be introduced as V₂O₅ dissolved in 5% by weight HCl.

The aqueous reagent of the present invention is particularly useful in an assay which is based on using the reduction of the tetrazolium salt, *e.g.,* 3-(4,5-dimethyl-2-thiazolyl)-2,5-diphenyl-2H-tetrazolium bromide (thiazolyl blue MTT) to a formazan (e.g., MTT-formazan) with phenazine methosulfate (PMS) as an electron transfer agent for detecting or quantifying the presence of a target molecule (or a fraction thereof), such as an antigen, an antibody or a polynucleotide, in a sample, particularly a sample of biological origin.

In this assay, an alkaline phosphatase (AP) conjugate acts as a reporter molecule and operates on a substrate {e.g., 5-bromo-4-chloroindoxyl phosphate (BCIP)} to produce an enzymatically altered substrate molecule. The enzymatically altered molecule, in turn, participates in oxidation-reduction reactions with a tetrazolium salt in the presence of the electron transfer agent (PMS) to cause the formation of a colored formazan species.

As a result, a particularly useful and preferred reagent composition of the present invention comprises an aqueous solution of PMS, nitric acid and a water soluble metal salt wherein the metal is selected from the group consisting of vanadium, manganese, cobalt, and mixtures thereof, in admixture with a dissolved tetrazolium salt and 5-bromo-4-chloroindoxyl phosphate (BCIP).

The tetrazolium salt is an organic salt in which the organic portion contains one or two tetrazole rings, generally with aryl (especially phenyl or substituted phenyl) substituents at various positions, particularly the 2, 3, and 5 positions. Tetrazolium salts with two tetrazole rings are typically coupled so as to provide a diphenyl group (theoretically using two of the previously mentioned phenyl substituents) with the tetrazole rings in the two para positions. The tetrazolium-containing organic component provides all or part of the positive charge of the salt with the negative charge being provided by an organic or inorganic negative ion, typically an inorganic halide ion. Exemplary tetrazolium compounds include, but are not limited to the following: Nitrotetrazolium Violet; p-Nitro Blue Tetrazolium Chloride; m-Nitro Blue Tetrazolium Chloride; [3-(4,5-Dimethylthiazol-2-yl)-2,5-diphenyl]tetrazolium Bromide (MTT); Iodo Nitro Tetrazolium Violet Chloride; 2,3,5-Triphenyl Tetrazolium Chloride; Distyryl Nitro Blue Tetrazolium Chloride (NTB); p-Anisyl Blue Tetrazolium Chloride; Tetranitro Blue Tetrazolium Chloride; 2,5-diphenyl-3-[4-naphthyl] -tetrazolium chloride; 2,2',5,5'-Tetraphenyl-3,3' (p- diphenylene)-ditetrazolium chloride; m-Nitro Neo-tetrazolium- Chloride; 2(2'-Benzothiazolyl) -5-styryl-3-(4'-phthalhydrazidyl)-tetrazolium chloride; 2,2'-di-p-nitrophenyl-5,5'-di-p-thiocarbamyl-phenyl-3,3'[3,3'-dimethoxy-4,4'-biphenylene] ditetrazolium chloride; o-Tolyl Tetrazolium Red; p-Tolyl Tetrazolium Red; Piperonyl Tetrazolium Blue; p-Anisyl-p-Nitro Blue Tetrazolium Chloride; Veratryl Tetrazolium Blue; and mixtures thereof. Of these, Nitrotetrazolium Violet, p-Nitro Blue Tetrazolium (NTB), MTT, and Iodonitro-tetrazolium (INT) salts are generally preferred.

When used for the determination of enzymes or organisms, the reagent composition of this invention preferably is buffered at the particular pH required for a given assay. Useful buffers can readily be determined by one skilled in the art and include, but are not limited to phosphates, borates and organic buffers such as reported by Good et al., in Biochem. 5, 467 (1966) and Anal. Biochem. 104, 300 (1980). One commonly used buffer is tris-HCl at a pH of 9.5 to 9.6

Preferably, the reagent also will usually contain a surfactant. Surfactants which are useful in the practice of this invention include, but are not limited to any surfactant which does not interfere with the enzymatic reactions and the redox reactions that occur during the use of the reagent composition. Generally, exemplary useful surfactants include anionic and nonionic surfactants. See, Remington's Pharmaceutical Sciences, 17th Edition, A.R. Gennaro, Editor, Mack Publishing Company, Easton, Pennsylvania (1985) for descriptions of various surfactants and other additives.

The terms "bound", "complexed", "attached" and words of similar import are intended throughout the specification and claims to include both covalent and non-covalent interactions, which may be direct or indirect, between two moieties or molecules. An indirect bond or interaction is one where one moiety is attached to another moiety through an intermediate moiety. Methods for forming a labeled detector molecule or probe, for example, an antibody (or fraction thereof) having alkaline phosphatase (AP) bound thereto, are well-known to those skilled in the art and thus require no exposition. Usually, the alkaline phosphatase is covalently bonded to the detector molecule or to an intermediate binding partner that can be complexed, directly or indirectly, to the detector molecule, by direct condensation or by using a known bridging molecule, such as a carbodiimide, a diisocyanate, a dialdehyde or the like.

The term "target material" refers to a material, such as a protein or other molecule (or fraction thereof, respectively), whose presence in a sample is to be detected, identified or quantified. In this regard, typical samples include blood, urine, other bodily fluids, and extracts of other samples.

In accordance with a more specific aspect of the present invention, 5-bromo-4-chloroindoxyl phosphate (BCIP) is used in the detection system as the alkaline phosphatase substrate. Without being bound by theory, BCIP is converted by alkaline phosphatase to the enol 5-bromo-4-chloroindoxyl which, in turn, tautomerizes under alkaline pH conditions to its keto form according to the following reactions (A) and (B): 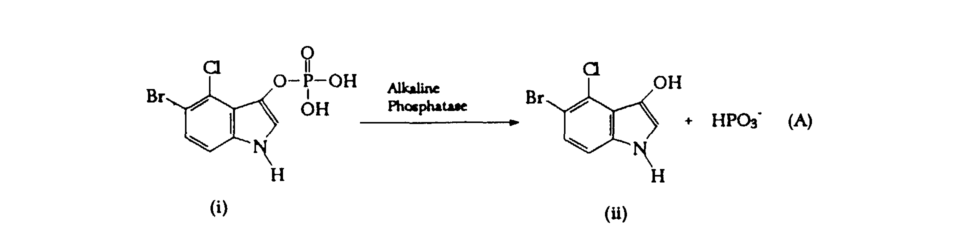 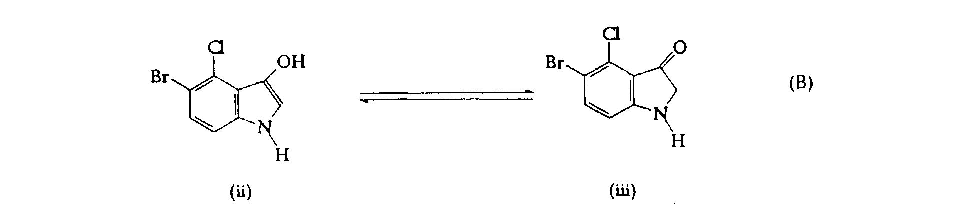 Further, without being bound by theory, it is believed that in accordance with the present invention, the keto species (iii) thereafter is trapped upon its conversion, by an oxidation to 5,5'-dibromo-4,4'-dichloro-indigo white (iv) through an oxidation/reduction (redox) reaction with a tetrazolium salt such as 3-(4,5-dimethyl-2-thiazolyl)-2, 5-diphenyl-2H-tetrazolium bromide (thiazolyl blue MTT) in the presence of PMS according to the following reaction (C): 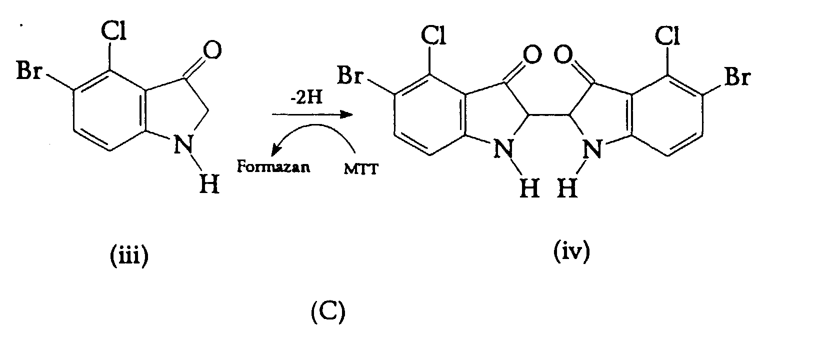 In the redox reaction (C), MTT is reduced to MTT-formazan having a blue color.

Another important aspect of the present invention involves including certain additional soluble divalent cations in the solution to improve the assay. In particular, certain divalent cations can be included in the reagent solution containing the tetrazolium salt to enhance the sensitivity of the assay. It has been determined that in the presence of certain metal cations, the absorbance peak of the formazan shifts about 50 nm towards the blue end of the visible spectra, but more importantly, the extinction coefficient is enhanced by about 40%. A divalent cation preferably is included in the reagent composition in a sensitivity enhancing amount of about 20% to about 200% by weight of the tetrazolium salt. As a result, commercially available colorimetric detection instrumentation, such as a Biotek Ceres 900 reader, can be used to detect the formazan chromophore with the required level of sensitivity. Divalent zinc (supplied as ZnO in 5% by weight nitric acid or 5% by weight hydrochloric acid) has been found to be a most suitable metal cation in this regard, although other divalent cations such as cadmium and copper also can be used. Cadmium may be provided as a divalent cation in the reagent of the present invention as an aqueous solution at an exemplary concentration of 10 ug/ml -> 200 ug/ml. Preferably, the aqueous solution of cadimium has a pH range from 5.5 -11.0. Copper nitrate, for example, helps to keep MTT in an oxidized state during storage. These cations also can be introduced into the assay as one of their commonly available water soluble salts, such as zinc chloride, zinc sulfate, zinc acetate, zinc bromide, zinc iodide, zinc propionate, zinc formate, zinc lactate, zinc nitrate, zinc citrate and the like and mixtures thereof.

As noted above, the alkaline phosphatase enzyme-labeled conjugate preferably is contacted with the reagent composition of the invention containing BCIP, MTT, PMS, nitric acid, a dissolved metal salt of a metal selected from the group consisting of vanadium, manganese, cobalt, and mixtures thereof, and optionally (i) a surfactant, and (ii) a sensitivity-enhancing amount of a divalent metal cation. After an appropriate period of time has elapsed for the various reactions (e.g., A, B and/or C) to take place, which may be for example from about 5 minutes to about 2 hours, the strength of the colored reaction product is measured. Detection, and preferably quantification, of the sample may be accomplished using any available instrument for assessing the strength of the chromogenic signal. Such analysis might be completed in several seconds and the results can be compared to the strength from a known reference sample as a way of quantifying the concentration of target molecule in the sample. Other ways of quantifying the results will be recognized by those skilled in the art and the invention is not limited to any particular assay method.

The all-in-one reagent of the present invention will typically be supplied as one component of a test pack or kit of reagents for conducting an immunoassay, a hybridization assay or other detection assay. In addition to the reagent composition of the present invention such kit will, in its simplest form, also contains one or more of the following components: (1) a quantity of one member of a pair of a ligand-receptor (e.g., antibody-antigen) complementary binding partners bound to a solid phase support; (2) a detector molecule bound to alkaline phosphatase, the detector molecule possibly being one member of the pair of ligand-receptor (e.g., antibody-antigen) complementary binding partners or a polynucleotide hybrid for the target molecule (enzyme conjugate); and possibly (3) an immunological binding partner of components (1) or (2).

The additional reagents used in connection with the reagent composition of the present invention also can take many forms. For example, the detector molecule reagent (e.g., a labeled antigen, a labeled antibody or a labeled polynucleotide) can be provided dissolved in an aqueous buffer solution, bound or impregnated on a solid support, such as a paper strip, or as a lyophilized powder.

The following example is provided for exemplification purposes only and is not intended to limit the scope of the invention which has been described in broad terms above.

### EXAMPLE 1

An all-in-one indicator reagent for conducting an assay which uses an alkaline phosphatase as the detector moiety or label was prepared as follows. To 9.345 ml of an aqueous buffer solution containing 0.1M Tris-HCl and 0.1M NaCl, and having a pH of 9.5 (which pH may be adjusted with NaOH) were added the following ingredients to yield a total final volume of about 10.0 ml:
(1) 20 µl of a manganese nitrate solution prepared by dissolving manganese to a concentration of 1000 ppm in 2% by weight nitric acid;
(2) 60 µl of a cobalt nitrate solution prepared by dissolving cobalt to a concentration of 1000 ppm in 2% by weight nitric acid;
(3) 250 µl of an aqueous solution containing MTT at a concentration of 10 mM;
(4) 100 µl of an aqueous solution containing PMS at a concentration of 10 mM;
(5) 75 µl of an aqueous solution of BCIP at a concentration of about 50 mg/ml; and
(6) 150 µl of a 10 wt. % solution of sodium dodecyl sulfate (SDS).
The resulting aqueous solution is stable against rapid discoloration and is useful as the indicator reagent in a formazan-based assay.

Thus, the invention which is intended to be protected herein is not to be construed as limited to the particular forms disclosed, since they are to be regarded as illustrative rather than restrictive. Variations and changes may be made by those skilled in the art without departing from the spirit and scope of the invention. For example, other adjuvants known to be useful in connection with an assay involving the reduction of a tetrazolium salt to a formazan can also be used as a component of or in combination with the aqueous PMS reagent of the present invention. Such adjuvants are recognized by those skilled in the art and do not require specific elaboration herein to described their utility. All references and patents cited herein are incorporated by reference in their entirety.

## Claims

1. A reagent composition for use in a tetrazolium-based assay which comprises:
(a) an aqueous solution comprising 5-methyl-phenazinium methyl sulfate, nitric acid and a metal salt wherein the metal is selected from the group consisting of vanadium, manganese, cobalt, and mixtures thereof.

2. A reagent composition for use in a tetrazolium-based assay which comprises:
(a) an aqueous solution comprising 5-methyl-phenazinium methyl sulfate, nitric acid, a water soluble metal salt wherein the metal is selected from the group consisting of vanadium, manganese, cobalt, and mixtures thereof; and
(b) a dissolved tetrazolium salt.

3. The reagent composition of claim 1, wherein said metal salt is selected from the group consisting of
(a) a metal chloride other than cobalt chloride, a metal sulfate, a metal phosphate, a metal nitrate and mixtures thereof.

4. The reagent composition of claim 3, wherein said metal salt is provided in a concentration sufficient to yield from about 0.5 to about 2.0 moles of said metal per mole of said 5-methyl-phenazinium sulfate in said reagent composition.

5. The reagent composition of claim 1, further comprising a reporter molecule.

6. The reagent composition of claim 5, wherein said reporter molecule is 5-bromo-4-chlorindoxyl phosphate.

7. The reagent composition of claim 1, further comprising at least one of a reporter molecule, a tetrazolium salt, a divalent cation, defoliant copper and mixtures thereof.

8. The reagent composition of claim 7, wherein said divalent cation is zinc.

9. The reagent composition of claim 8, wherein said zinc is provided as a water soluble salt selected from the group consisting of zinc chloride, zinc sulfate, zinc acetate, zinc bromide, zinc iodide, zinc propionate, zinc formate, zinc lactate, zinc nitrate, zinc citrate and mixtures thereof.

10. The reagent composition of claim 7, wherein said divalent metal cation is a divalent metal salt, wherein said cation is associated with an anion selected from the group consisting of a chloride, a sulfate, an acetate, a bromide, an iodide, a propionate, a formate, a lactate, a nitrate, a citrate and mixtures thereof.

11. The reagent composition of claim 7, further comprising a surfactant.

12. The reagent composition of claim 11, wherein said surfactant is an anionic surfactant or an non-ionic surfactant.

13. The reagent composition of claim 12, wherein said surfactant is an anionic surfactant.

14. The reagent composition of claim 12, wherein said surfactant is sodium dodecyl sulfate.

15. The reagent composition of claim 2, wherein said tretrazolium salt is selected from the group consisting of nitrotetrazolium violet, p-nitro blue tetrazolium chloride, m-nitro blue tetrazolium chloride, (3-(4,5- dimethylthiazol-2-yl) -2,5-diphenyl)tetrazolium bromide, iodo nitro tetrazolium violet chloride, 2,3,5-triphenyl tetrazolium chloride, distyryl nitro blue tetrazolium chloride, p-anisyl blue tetrazolium chloride, tetranitro blue tetrazolium chloride, 2,5-diphenyl-3-(4-naphthyl)-tetrazolium chloride, 2,2',5,5'-tetraphenyl-3,3' (p-diphenylene)-ditetrazolium chloride, m-nitro neo-tetrazolium-chloride,2-(2'-benzothiazolyl)-5-styryl-3-(4'-phthalhydrazidyl)-tetrazolium chloride, 2,2'-di-p-nitrophenyl-5,5'- di-p-thiocarbamyl -phenyl- 3,3'(3,3' -dimethoxy -4, 4'- biphenylene) ditetrazolium chloride, o-tolyl tetrazolium red, p-tolyl tetrazolium red, piperonyl tetrazolium blue, p-anisyl-p-nitro blue tetrazolium chloride, veratryl tetrazolium blue and mixtures thereof.

16. The reagent composition of claim 1, wherein said reagent composition has a pH from about 5.0 to about 11.0.

17. The reagent composition of claim 16, wherein said pH is from about 9.5 to about 9.6.

18. The reagent composition of claim 1 further comprising a divalent metal cation selected from Zn⁺², Cd⁺², Cu⁺² and mixtures thereof.

19. The reagent composition of claim 2 further comprising a divalent metal cation selected from the group consisting of Zn⁺², Cd⁺², Cu⁺² and mixtures thereof.

20. The reagent composition of claim 3 further comprising a divalent metal cation selected from the group consisting of Zn⁺², Cd⁺², Cu⁺² and mixtures thereof.

21. The reagent composition of claim 19 wherein the tetrazolium salt is selected from nitrotetrazolium violet, p-nitro blue tetrazolium chloride, m-nitro blue tetrazolium chloride, (3-(4,5-Dimethylthiazol-2-yl)-2,5-diphenyl)tetrazolium bromide, iodo nitro tetrazolium violet chloride, 2,3,5-triphenyl tetrazolium chloride, distyryl nitro blue tetrazolium chloride, p-anisyl blue tetrazolium chloride, tetranitro blue tetrazolium chloride, 2,5-diphenyl-3-(4-naphthyl) -tetrazolium chloride, 2,2',5,5'-tetraphenyl-3,3' (p- diphenylene)-ditetrazolium chloride, m-nitro neo-tetrazolium- chloride, 2-(2'-benzothiazolyl)-5-styryl-3 -(4'- phthalhydrazidyl) -tetrazolium chloride, 2,2'-di-p- nitrophenyl- 5,5'-di-p-thiocarbamyl- phenyl-3,3' (3,3'-dimethoxy-4,4'-biphenylene) ditetrazolium chloride, o-tolyl tetrazolium red, p-tolyl tetrazolium red, piperonyl tetrazolium blue, p-anisyl-p-nitro blue tetrazolium chloride, veratryl tetrazolium blue and mixtures thereof.
